# EUROPÄISCHE PATENTSCHRIFT

(11) **EP 3 487 552 B1**
(45) Veröffentlichungstag und Bekanntmachung des Hinweises auf die Patenterteilung: **05.11.2025**
(21) Anmeldenummer: 17742373.8
(22) Anmeldetag: 20.07.2017
(51) Int. Cl.: A61M 1/14, A61M 1/28, A61M 1/16

(54) **VERFAHREN ZUR BELADUNG EINES MEDIZINISCHEN GERÄTES**
METHOD FOR LOADING A MEDICAL DEVICE
PROCÉDÉ DE CHARGE D'UN APPAREIL MÉDICAL

(30) Priorität: 20.07.2016 DE 102016008868
(43) Veröffentlichungstag der Anmeldung: 29.05.2019
(73) Patentinhaber: Fresenius Medical Care Deutschland GmbH, 61352 Bad Homburg (DE)
(72) Erfinder: GRIEßMANN, Erik, 97422 Schweinfurt (DE); WOLF, Klaus, 97450 Arnstein (DE); WABEL, Peter, 64288 Darmstadt (DE); MOHR, Bernd, 64295 Darmstadt (DE)
(74) Vertreter: Herrmann, Uwe
(86) Internationale Anmeldenummer: PCT/EP2017/000886
(87) Internationale Veröffentlichungsnummer: WO 2018/015019

(56) Entgegenhaltungen:
- EP-A1- 0 846 470
- US-A1- 2010 315 231
- US-A1- 2011 315 611
- US-A1- 2016 022 893

## Beschreibung

Die Erfindung betrifft ein Verfahren zur Beladung eines medizinischen Gerätes, insbesondere eines Dialysegeräts und vorzugsweise eines Peritonealdialysegeräts sowie ein medizinisches Gerät, insbesondere Dialysegerät, wie z.B. ein Peritonealdialysegerät zur Durchführung eines derartigen Verfahrens.

Vor Durchführung einer Peritonealdialysebehandlung muss sich der Patient selbständig mit dem Peritonealdiaysegerät verbinden und auch verschiedene Flüssigkeitsbehältnisse in ausreichender Menge an das Gerät anschließen. Bei unsachgemäßer Handhabung können Fehler auftreten, was aber unbedingt zu vermeiden ist, da ansonsten die Behandlung nicht durchgeführt werden darf.

Aus der US2011/315611A1 ist beispielsweise ein portables Dialysegerät bekannt. Zudem ist aus der US2010/0315231A1 ein Gerät zur extrakorporalen Blutbehandlung bekannt. Weitere Dokumente des Stands der Technik sind EP0846470A1 und US2016/022893A1.

Aufgabe der Erfindung ist es, ein verbessertes Verfahren zur Durchführung und Überprüfung des Aufrüstvorgangs einer Dialysebehandlung bereitzustellen.

Diese Aufgabe wird durch die Gegenstände der unabhängigen Ansprüche gelöst. Vorteilhafte Ausgestaltungen der Erfindung sind Gegenstand der abhängigen Ansprüche.

Vor diesem Hintergrund betrifft die Erfindung ein Verfahren zur Beladung eines medizinischen Gerätes, z.B. eines Dialysegeräts und vorzugsweise eines Peritonealdialysegeräts, das eine Gerätesteuerung und ein Benutzerinterface aufweist, wobei mehrere fluidgefüllte Behältnisse sequentiell an entsprechende Schnittstellen des Geräts angeschlossen werden und für alle Behältnisse vor oder bei deren Anschluss eine und vorzugsweise dieselbe Eigenschaft geprüft und die Messwerte jeweils mit wenigstens einem in der Gerätesteuerung hinterlegten Sollwert verglichen werden. Weiterhin wird das Gewicht eines jeden Behältnisses gegen eine hinterlegte Liste möglicher Behältnisvolumina abgeglichen, wobei jedes Behältnisvolumen durch eine untere und obere Toleranzgrenze bestimmt ist und ein Überfüllungvolumen umfassen kann, und es erfolgt ein Plausibilitätscheck, in dem abgefragt wird, ob eine Dialyselösung existiert, die zu der verwendeten Behältniskombination korrespondiert.

Im Folgenden verwendete Bezugnahmen auf Dialyse- oder Peritonealdialysegeräte gelten entsprechend allgemein auch für medizinische Geräte.

Dabei können die während der Aufrüstphase aufgelegten Behältnisse hinsichtlich der enthaltenen Volumina und/oder mit der patientenindividuellen Therapieverschreibung auf Plausibilität geprüft werden. Dies trägt insbesondere im Heimdialysebereich zu einer verbesserten Patientensicherheit bei, da mögliche Fehlbeladungen oder Fehlanschlüsse durch den Patienten aufgrund der Plausibilitätsprüfung erkannt werden können und das vorgeschlagene Verfahren in einer Ausführungsform gleichzeitig auch eine Anwenderführung umfasst.

Die Behältnisse werden vor oder bei deren Anschluss gewogen. Bei der geprüften Eigenschaft der Behältnisse handelt es sich also um das Gewicht des Behältnisses, das mit wenigstens einem im Gerät hinterlegten Sollwert für das Gewicht verglichen wird.

In einer Ausführungsform wird am Benutzerinterface angezeigt, ob der Messwert in dem Sollwertbereich liegt oder innerhalb vorgegebener Toleranzgrenzen liegt.

Alternativ kann dies auch über einen anderen Indikator als das Gewicht erfolgen, wie beispielsweise über das Volumen oder auch über die Lösungsart etc.

In einer Ausführungsform werden ein Anschluss des Behältnisses und/oder nachfolgende Schritte von der Gerätesteuerung gesperrt, wenn der Messwert nicht in dem Sollwertbereich liegt oder innerhalb vorgegebener Toleranzgrenzen liegt.

Alternativ oder zusätzlich kann ein entsprechender Hinweis an den Anwender ausgegeben werden.

Erfindungsgemäß werden mehrere fluidgefüllte Behältnisse sequentiell an entsprechende Schnittstellen des Geräts angeschlossen und für alle Behältnisse vor oder bei deren Anschluss eine und vorzugsweise dieselbe Eigenschaft geprüft und die Messwerte jeweils mit wenigstens einem in der Gerätesteuerung hinterlegten Sollwertbereich verglichen. Beispielsweise kann vorgesehen sein, dass mehrere Behältnisse nacheinander auf dieselbe Waage gelegt werden und bei Hinzufügen eines neuen Behältnisses jeweils die Gewichtsdifferenz gemessen wird.

Anstelle von mehreren Behältnissen können aber auch Lösungsvolumina verschrieben werden, welche mit einer Beutelkombination erfüllt werden müssen. Sind z.B. für eine Therapie z.B. 10.000 ml verschrieben, so können beispielweise 2 x 5.000 ml oder 2 x 6.000 ml verwendet werden, um die Verschreibung zu erreichen. Dies ist insbesondere für den Fall relevant, dass eine Lösungsart in verschiedenen Verpackungsgrößen vorliegt.

Bei den Behältnissen kann es sich beispielsweise um Beutel handeln.

In einer Ausführungsform wird der Sollwertbereich von der Gerätesteuerung aufgrund gespeicherter Werte für jeweilige Eigenschaften bekannter Behältnisse und/oder aufgrund einer gespeicherten ärztlichen Verschreibung festgelegt.

In einer Ausführungsform ist vorgesehen, dass die Behältnisse an ihrem Messplatz angeschlossen werden. Beispielsweise wird die Messung an einer Heiz- und Wägeschale des Dialysegerätes durchgeführt, in der die angeschlossenen Behältnisse sich auch während einer nachfolgenden Behandlung befinden. Alternativ kann als Messplatz auch die Drainschale vorgesehen sein, da ebenfalls das Gewicht der Drainschale erfasst wird. Wird hierbei ein zu hohes Gewicht erkannt, so kann ein Hinweis erfolgen, dass der Drainbag oder -Container voll ist und/oder geleert werden soll.

Weiterhin kann mit dem vorgeschlagenen Verfahren ein Funktionstest (z.B. Kalibrierung) des Wägesystems durchgeführt werden und/oder eine mögliche Fehlfunktion erkannt werden.

Vor dem eingangs genannten Hintergrund betrifft die Erfindung ferner ein medizinisches Gerät, insbesondere ein Dialysegerät und vorzugsweise ein Peritonealdialysegerät mit einer Gerätesteuerung, einem Benutzerinterface und Schnittstellen zum Anschluss fluidgefüllter Behältnisse, wobei das Gerät eine Messeinrichtung zur Überprüfung wenigstens einer Eigenschaft des Behältnisses aufweist und wobei die Gerätesteuerung ausgebildet ist, ein Verfahren nach einem der vorhergehenden Ansprüche durchzuführen.

In einer Ausführungsform handelt es sich bei der Messeinrichtung um eine Waage. In einer Ausführungsform ist eine gemeinsame Messeinrichtung für alle Behältnisse vorhanden. Der Messplatz kann dem finalen Anschlussplatz der Behältnisse entsprechen.

Bei der Messeinrichtung kann es sich beispielsweise um eine Heiz-, Drain- und Wägeschale des Dialysegeräts handeln.

In einer Ausführungsform ist die Gerätesteuerung ferner ausgebildet, eine Eigenschaft eines weiteren Behältnisses, das während oder am Ende einer Behandlung an das Gerät angeschlossen wird, vor oder bei dessen Anschluss zu prüfen und den Messwert mit wenigstens einem in der Gerätesteuerung hinterlegten Sollwert zu vergleichen. Das erfinderische Prinzip kann in dieser Ausführungsform also nicht nur in der Vorbereitungsphase Anwendung finden, sondern zusätzlich auch dann, wenn während oder am Ende einer Behandlung der Anschluss eines weiteren Behältnisses notwendig ist.

Die Erfindung betrifft also das sequentielle Beladen der Heiz- und/oder Wägeschale eines Dialysegerätes, insbesondere eines Peritonealdialysegerätes mit Dialysefluid gefüllten Behältnissen und eine Plausibilitätsprüfung der aufgelegten Behältnisse.

In einer weiteren Ausführungsform kann die Heizung auch unabhängig vom Gerät angeordnet sein.

Weitere Einzelheiten und Vorteile der Erfindung ergeben sich aus dem nachfolgend beschriebenen Ausführungsbeispiel und aus den Figuren. In den Figuren zeigen:
- Figur 1:: eine schematische Darstellung eines erfindungsgemäßen Verfahrensablaufs; und
- Figur 2:: eine schematische Darstellung des Verlaufs der Messwerte für das Gewicht der angeschlossenen Beutel.

Ein erfindungsgemäßes Dialysegerät gemäß Ausführungsbeispiel ist mit einer Heiz- und/oder Wägeschale ausgestattet und verfügt über eine Benutzerführung, die den Anwender/Patienten durch den Vorbereitungsprozess seiner Behandlung führt und in einem internen Prozess die aufgelegten Behältnisse auf ihre Plausibilität für die vorgesehene Behandlung überprüft werden können.

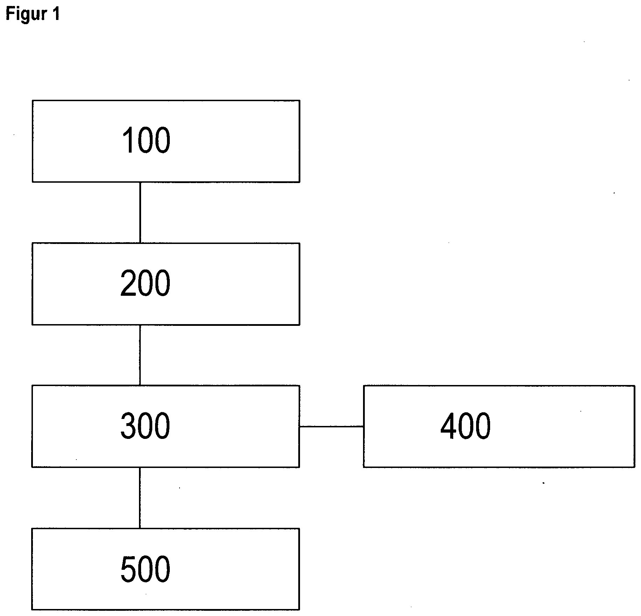

Figur 1 zeigt eine schematische Darstellung eines möglichen Verfahrens zur Beladung eines Dialysegeräts gemäß Ausführungsbeispiel.

Dabei wird der Anwender in einem ersten Schritt 100 durch das Benutzerinterface aufgefordert, zu prüfen, dass sich keine Gegenstände, insbesondere Behältnisse auf der Heiz-und/oder Wägeschale und der Drainschale befinden.

Den Zustand der unbeladenen Heiz-und/oder Wägeschale und der Drainschale legt das Wägesystem als Nullpunkt fest. Dabei kann für den Nullpunkt ein Toleranzband im Bereich zwischen +/-500 bis +/-20 g, vorzugsweise im Bereich von +/-300 bis +/-100 g und besonders bevorzugt von +/-200 bis +/-150 g vorgesehen sein.

Im Anschluss daran wird der Anwender bzw. Patient über das Benutzerinterface in einem weiteren Schritt 200 aufgefordert, das erste Behältnis auf die Heiz-und/oder Wägeschale aufzulegen. Als Behältnisse werden vorzugsweise Folienbeutel verwendet.

Das Gerät erkennt das Gewicht des Beutels und gleicht dieses gegen eine im Gerät hinterlegte Liste möglicher Beutelvolumina ab. Eine Stabilisierungsphase, die beispielsweise 2 Sekunden ab Beutelauflage dauern kann, verbessert die Messgenauigkeit. Da das Material des Behältnisses sowie eine produktionsbedingte Überfüllung mit Dialysefluid in die Gewichtsmessung miteingehen kann, ist auch hier ein Toleranzbereich in Anhängigkeit von den vorgesehen Beutelvolumina vorgesehen. Beispiele für geeignete beutelgrößenabhängige Toleranzgrenzen werden in der nachfolgenden Tabelle 1 genannt.

**Tabelle 1**

| Beutelvolumen | Untere Toleranzgrenze | Obere Toleranzgrenze |
|---|---|---|
| 2.000 ml | 2.000 g | 2.500 g |
| 2.500 ml | 2.500 g | 3.000 g |
| 5.000 ml | 5.000 g | 5.600 g |
| 6.000 ml | 6.000 g | 6.600 g |

Gleichzeitig prüft das Gerät, ob das erkannte Beutelvolumen auch mit der entsprechenden Therapieverschreibung des Patienten korrespondiert. Erkennt das Gerät beide Kriterien (Behältnis mit korrektem Füllvolumen wurde aufgelegt und stimmt mit Verschreibung überein) als erfüllt an, so wird der Patient bzw. Anwender aufgefordert, das nächste Behältnis aufzulegen. Die Plausibilitätsprüfung für den zweiten und für weitere Beutel wird ebenfalls wie oben beschrieben durchgeführt.

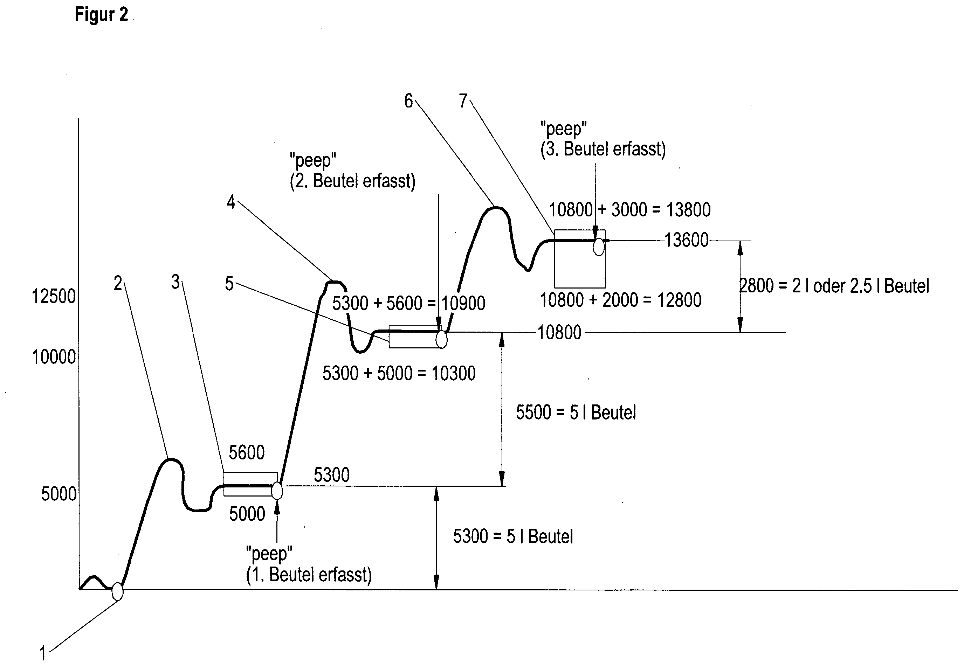

Figur 2 zeigt eine schematische Darstellung der Entwicklung des Gewichtssignals bei einer Auflage mehrerer Beutel. Der Nullpunkt ist mit dem Bezugszeichen 1 markiert. Sodann wird der erste Beutel aufgelegt. Nach einem schwankenden Messsignal in einer Stabilisierungsphase 2 wird während einer Messphase 3 ein Gewicht von 5.300 g erkannt. Dieses Gewicht wird einem Beutelvolumen von 5.000 ml zugeordnet und mit Bezug auf die gespeicherte Therapieverordnung als plausibel eingestuft. Sodann wird 5.300 g als neuer Referenzpunkt angenommen und es wird ein zweiter Beutel aufgelegt. Nach einem schwankenden Messsignal in einer zweiten Stabilisierungsphase 4 wird während einer zweiten Messphase 5 ein Gewicht von 5.500 g erkannt. Auch dieses Gewicht wird einem Beutelvolumen von 5.000 ml zugeordnet und als mit Bezug auf die gespeicherte Therapieverordnung stimmig eingestuft. Nun wird das bisherige Gesamtgewicht von 10.800 g als neuer Referenzpunkt angenommen und es wird ein dritter Beutel aufgelegt. Nach einem schwankenden Messsignal in einer dritten Stabilisierungsphase 6 wird während einer dritten Messphase 7 ein Gewicht von 2.800 g erkannt. Dieses Gewicht kann zu einem Beutelvolumen von 2.000 ml oder 2.500 ml korrespondieren, wobei eines der beiden Volumina mit der gespeicherten Therapieverordnung übereinstimmt und daher als insgesamt stimmig erkannt wird.

Sind für eine Verschreibung z.B. nur zwei Beutel vorgesehen, ist nach dem Laden des zweiten Beutels das Gesamtgewicht erreicht und die Sequenz wird automatisch beendet. Der Anwender wird dann nicht aufgefordert, einen weiteren Beutel aufzulegen.

Gemäß einem weiteren Schritt 300 kann ein Plausibilitätscheck erfolgen, in dem abgefragt wird, ob eine Dialyselösung existiert, die zu der verwendeten Beutelkombination korrespondiert. Ferner kann abgefragt werden, ob ein Maximalvolumen von insgesamt 14.500 ml nicht überschritten wird und/oder ob eine Maximalanzahl an unterschiedlichen Beuteln von 3 nicht überschritten wird.

Beispielsweise kann eine Lösungsart mit nur einer bestimmten Konzentration verschrieben werden, die nur in einer für die Lösungsart korrespondierenden Beutelgröße verfügbar ist. Wird eine andere Beutelgröße oder -volumen aufgelegt, kann daraus erkannt werden, dass nicht die korrekte Lösungsart oder der korrekte Lösungsbeutel aufgelegt wurde. Für eine Verschreibung können auch weniger als die vorhandenen Behandlungsplätze verwendet werden.

Misst das Gerät ein Gewicht, das keinem plausiblen vorgegebenen Beutelvolumen entspricht bzw. nicht mit der Therapieverschreibung im Einklang ist, wird in einem Schritt 400 eine Fehlermeldung ausgegeben, die den Anwender bzw. Patienten auffordert, das korrekte Behältnis aufzulegen. Erst nach der automatischen Überprüfung der Fehlerbehebung kann mit dem Aufrüsten des Gerätes fortgefahren werden.

Bei erfolgreicher Beladung wird in einem Schritt 500 die Therapie freigegeben.

Optional kann in der Therapieverschreibung am Ende der Behandlung eine letzte Befüllung des Patienten mit Dialysat vorgesehen sein. Hierfür sieht die Verschreibung als weiteren Schritt die Auflage eines letzten Beutels vor. Auch hier erkennt das Gerät, ob der Anwender bzw. Patient gemäß der Verschreibung diesen letzten Beutel aufgelegt hat. In diesem Fall wird der Ablauf - ggf. auf einer graphischen Nutzeroberfläche - verändert und eine weitere Klemme zugeschaltet.

Entsprechend erkennt das Gerät ebenfalls wenn ein zusätzlicher Beutel aufgelegt wurde, der laut Verschreibung nicht vorgesehen ist und fordert den Anwender bzw. Patienten auf, diesen von der Heiz- und/oder Wägeschale zu entfernen.

Sofern bei einem Fehleralarm durch den Anwender bzw. Patienten sichergestellt werden kann, dass alle Beutel korrekt aufgelegt wurden und auch mit der Verschreibung übereinstimmen, der Fehleralarm aber immer noch angezeigt wird, liefert dies einen Hinweis, dass es sich um einen Defekt am Wägesystem handeln kann.

Ein weiterer Vorteil besteht darin, dass mit diesem Verfahren alle für die Behandlung verwendeten Dialysatbeutel auf ihre Plausibilität hinsichtlich der aufgelegten Beutelvolumina und der Verschreibung geprüft werden können und somit eine erhöhte Therapiesicherheit bieten.

Des Weiteren erlaubt das Verfahren eine Anwenderführung, die den Anwender bzw. Patienten Schritt für Schritt durch den Aufrüstungsprozess leitet und so einen wesentlichen Beitrag zur Patientensicherheit darstellt.

Da sowohl an der Heiz- und/oder Wägeschale als auch an der Drainschale Halteelemente in Form von Haltebügeln und/oder aufsteckbaren Seitenwänden angebracht werden müssen, um ein Abrutschen der Behältnisse zu vermeiden, kann über das ermittelte Gewicht in einer Ausgestaltung der Erfindung auch kontrolliert werden, ob diese sicherheitsrelevanten Teile montiert sind. Dies setzt voraus, dass die Gewichte für die fertig montierte Heiz- und Drainschale im Gerät hinterlegt sind.

In einer Ausführungsform ist es auch möglich, mittels einer gesteuerten Überfüllung der Dialysatbeutel auf einfache Art eine Identifizierung des Lösungstyps vorzunehmen. Hierzu kann für jeden einzelnen Lösungstyp eine produktionsbedingte Überfüllung über das Nominalvolumen der Beutel festgelegt werden. Das so für jeden Lösungstyp spezifische Gewicht kann ebenfalls in einer Tabelle für den Plausibilitätsabgleich hinterlegt werden. Ebenso kann auch hier ein Toleranzband für jeden Lösungstyp vorgesehen sein.

Gleichzeitig kann das Überfüllungsvolumen mittels des Wägesystems erfasst werden und als Therapievolumen bei der Behandlung mitverwendet werden. Dies bringt deutliche Vorteile, da das komplette Volumen in den Beuteln einschließlich des Überfüllungsvolumens für die Therapie genutzt werden kann.

Darüber hinaus kann das produktionsbedingte Füllgewicht für jeden einzelnen Lösungstyp im Gerät hinterlegt sein und beispielsweise mit einem Produktionsdatum, Chargen- oder Batch-Nr. verknüpft werden, welches zur näheren Spezifizierung des Dialysefluids herangezogen werden kann. Denkbar ist auch, dass lösungs- oder produktionsspezifische Angaben händisch eingegeben werden oder mittels Smartphone z.B. durch Auslesen eines QR-Codes oder Barcodes oder durch Nahfeldkommunikation oder per Cloud an das Gerät übermittelt werden können.

Damit können auch fehlerhafte Beutel erkannt werden. Beispielsweise Beutel, die aufgrund des Produktionsdatums als verfallen erkannt werden oder Beutel, die nicht dem spezifizierten Füllgewicht entsprechen, da sie beispielsweise bei zu hoher Temperatur gelagert wurden und somit ein zu geringes Gewicht aufweisen.

## Patentansprüche

1. Verfahren zur Beladung eines medizinischen Gerätes, inbesondere eines Dialysegeräts und vorzugsweise eines Peritonealdialysegeräts, das eine Gerätesteuerung und ein Benutzerinterface aufweist, wobei mehrere fluidgefüllte Behältnisse sequentiell an entsprechende Schnittstellen des Geräts angeschlossen werden und für alle Behältnisse vor oder bei deren Anschluss eine und vorzugsweise dieselbe Eigenschaft geprüft und die Messwerte jeweils mit wenigstens einem in der Gerätesteuerung hinterlegten Sollwert verglichen werden, wobei ein jedes Behältnis bei dessen Anschluss gewogen wird,
**dadurch gekennzeichnet, dass**
das Gewicht eines jeden Behältnisses gegen eine hinterlegte Liste möglicher Behältnisvolumina abgeglichen wird, wobei jedes Behältnisvolumen durch eine untere und obere Toleranzgrenze bestimmt ist und ein Überfüllungvolumen umfassen kann, und ein Plausibilitätscheck erfolgt, in dem abgefragt wird, ob eine Dialyselösung existiert, die zu der verwendeten Behältniskombination korrespondiert.

2. Verfahren nach einem der vorhergehenden Ansprüche, **dadurch gekennzeichnet, dass** am Benutzerinterface angezeigt wird, ob der Messwert dem Sollwert entspricht oder innerhalb vorgegebener Toleranzgrenzen liegt.

3. Verfahren nach einem der vorhergehenden Ansprüche, **dadurch gekennzeichnet, dass** ein Anschluss des Behältnisses und/oder nachfolgende Schritte von der Gerätesteuerung gesperrt werden, wenn der Messwert nicht dem Sollwert entspricht oder innerhalb vorgegebener Toleranzgrenzen liegt.

4. Verfahren nach einem der vorhergehenden Ansprüche, **dadurch gekennzeichnet, dass** der Sollwert von der Gerätesteuerung aufgrund gespeicherter Werte für jeweilige Eigenschaften bekannter Behältnisse und/oder aufgrund einer gespeicherten ärztlichen Verschreibung festgelegt wird.

5. Medizinisches Gerät, insbesondere Dialysegerät und vorzugsweise Peritonealdialysegerät mit einer Gerätesteuerung, einem Benutzerinterface und Schnittstellen zum Anschluss fluidgefüllter Behältnisse,
**dadurch gekennzeichnet,**
**dass** das Gerät eine Messeinrichtung zur Überprüfung wenigstens einer Eigenschaft der Behältnisse aufweist und dass die Gerätesteuerung ausgebildet ist, ein Verfahren nach einem der vorhergehenden Ansprüche durchzuführen.

6. Medizinisches Gerät nach Anspruch 5, **dadurch gekennzeichnet, dass** es sich bei der Messeinrichtung um eine Waage handelt.

7. Medizinisches Gerät nach Anspruch 5 oder 6, **dadurch gekennzeichnet, dass** eine gemeinsame Messeinrichtung für alle Behältnisse vorhanden ist und/oder dass der Messplatz dem finalen Anschlussplatz der Behältnisse entspricht.

8. Medizinisches Gerät nach einem der Ansprüche 5 bis 7, **dadurch gekennzeichnet, dass** die Gerätesteuerung ferner ausgebildet ist, eine Eigenschaft eines weiteren Behältnisses, das während oder am Ende einer Behandlung an das Gerät angeschlossen wird, vor oder bei dessen Anschluss zu prüfen und den Messwert mit wenigstens einem in der Gerätesteuerung hinterlegten Sollwert zu vergleichen.

## Claims

1. A method of loading a medical device, in particular a dialysis device, and preferably a peritoneal dialysis device, which comprises a device control and a user interface, wherein at least one fluid-filled container is connected to a corresponding interface of the device and for all containers one and preferably the same property is checked before or during its connection and the measured values are compared with at least one target value stored in the device control, wherein the container is weighed during its connection,
**characterized in that**
the weight of each container is compared with a stored list of potential container columina, wherein each container volume is determined by a lower and an upper tolerance limit and may include an overflow volume, and a plausibility check is carried out in which it is inquired whether a dialysis solution exists which corresponds to the container combination in use.

2. The method in accordance with any one of the preceding claims, **characterized in that** it is displayed at the user interface whether the measured value corresponds to the desired value or lies within predefined tolerance limits.

3. The method in accordance with any one of the preceding claims, **characterized in that** a connection of the container and/or subsequent steps are blocked by the device control if the measured value does not correspond to the desired value or does not lie within predefined tolerance limits.

4. The method in accordance with any one of the preceding claims, **characterized in that** the desired value is set by the device control on the basis of stored values for respective properties of known containers and/or is set on the basis of a stored medical prescription.

5. A medical device, in particular a dialysis device, and preferably a peritoneal dialysis device, comprising a device control, a user interface and interfaces for connecting fluid-filled containers,
**characterized in that**
the device comprises a measuring unit for checking at least one property of the containers; and **in that** the device control is configured to carry out a method in accordance with any one of the preceding claims.

6. The medical device in accordance with claim 5, **characterized in that** the measuring unit is a scale.

7. The medical device in accordance with claim 5 or 6, **characterized in that** a common measuring unit is present for all containers, and/or **in that** the measurement position corresponds to the final connection position of the containers.

8. The medical device in accordance with any one of the claims 5 to 7, **characterized in that** the device control is furthermore configured to check a property of a further container, which is connected to the device during or at the end of a treatment, before or during its connection and to compare the measured value with at least one desired value stored in the device control.

## Revendications

1. Procédé de chargement d'un appareil médical, notamment d'un appareil de dialyse et de préférence d'un appareil de dialyse péritonéale, qui présente une commande d'appareil et une interface utilisateur, plusieurs récipients remplis de fluide étant raccordés de manière séquentielle à des interfaces correspondantes de l'appareil et une et de préférence la même propriété étant contrôlée pour tous les récipients avant ou lors de leur raccordement et les valeurs de mesure étant comparées respectivement à au moins une valeur de consigne mémorisée dans la commande d'appareil, chaque récipient étant pesé lors de son raccordement,
**caractérisé en ce que**
le poids de chaque récipient est comparé à une liste mémorisée de volumes de récipient possibles, chaque volume de récipient étant déterminé par une limite de tolérance inférieure et supérieure et pouvant comprendre un volume de trop-plein, et une vérification de plausibilité est effectué, dans laquelle il est demandé s'il existe une solution de dialyse qui correspond à la combinaison de récipients utilisée.

2. Procédé selon l'une quelconque des revendications précédentes, **caractérisé en ce que** l'interface utilisateur indique si la valeur de mesure correspond à la valeur de consigne ou se situe dans des limites de tolérance prédéfinies.

3. Procédé selon l'une quelconque des revendications précédentes, **caractérisé en ce qu'**un raccordement du récipient et/ou des étapes ultérieures sont bloqués par la commande d'appareil si la valeur de mesure ne correspond pas à la valeur de consigne ou ne se situe pas dans des limites de tolérance prédéfinies.

4. Procédé selon l'une quelconque des revendications précédentes, **caractérisé en ce que** la valeur de consigne est fixée par la commande d'appareil sur la base de valeurs mémorisées pour des propriétés respectives de récipients connus et/ou sur la base d'une prescription médicale mémorisée.

5. Appareil médical, notamment appareil de dialyse et de préférence appareil de dialyse péritonéale avec une commande d'appareil, une interface utilisateur et des interfaces pour le raccordement de récipients remplis de fluide,
**caractérisé en ce que**
l'appareil présente un dispositif de mesure pour contrôler au moins une propriété des récipients et **en ce que** la commande d'appareil est réalisée pour mettre en œuvre un procédé selon l'une quelconque des revendications précédentes.

6. Appareil médical selon la revendication 5, **caractérisé en ce que** le dispositif de mesure consiste en une balance.

7. Appareil médical selon la revendication 5 ou 6, **caractérisé en ce qu'**un dispositif de mesure commun à tous les récipients est présent et/ou **en ce que** l'emplacement de mesure correspond à l'emplacement de raccordement final des récipients.

8. Appareil médical selon l'une quelconque des revendications 5 à 7, **caractérisé en ce que** la commande d'appareil est en outre réalisée pour contrôler une caractéristique d'un autre récipient qui est raccordé à l'appareil pendant ou à la fin d'un traitement, avant ou lors de son raccordement, et pour comparer la valeur de mesure à au moins une valeur de consigne mémorisée dans la commande d'appareil.
